# EUROPEAN PATENT SPECIFICATION

(11) **EP 2 058 010 B1**
(45) Date of publication and mention of the grant of the patent: **02.05.2012**
(21) Application number: 07805922.7
(22) Date of filing: 09.08.2007
(51) Int. Cl.: A61K 9/20, A61K 31/4245

(54) **PHARMACEUTICAL COMPOSITION**
PHARMAZEUTISCHE ZUSAMMENSETZUNG
COMPOSITION PHARMACEUTIQUE

(30) Priority: 10.08.2006 JP 2006218145
(43) Date of publication of application: 13.05.2009
(73) Proprietor: Takeda Pharmaceutical Company Limited, Osaka-shi, Osaka 541-0045 (JP)
(72) Inventor: NOMURA, Yukihiro, Osaka-shi, Osaka 532-8686 (JP); NONOMURA, Muneo, Osaka-shi, Osaka 532-8686 (JP)
(74) Representative: Jones, Nicholas Andrew
(86) International application number: PCT/JP2007/065666
(87) International publication number: WO 2008/018569

(56) References cited:
- EP-A- 0 546 358
- WO-A1-2004/078212
- JP-A- 03 086 830
- JP-A- 05 194 218
- JP-A- 11 035 451
- JP-A- 11 315 034
- JP-A- 2000 053 563
- JP-A- 2000 281 564
- JP-A- 2005 220 024
- KIBBE A H ET AL: "Handbook of Pharmaceutical Excipients" 2000, APHA & PHP , WASHINGTON (US) , XP002540628 page 244, paragraph entitled "7. Applications in Pharmaceutical Formulation or Technology"
- NAKANO M ET AL: "Sustained release of theophylline from hydroxypropylcellulose tablets." JOURNAL OF PHARMACEUTICAL SCIENCES APR 1983, vol. 72, no. 4, April 1983 (1983-04), pages 378-380, XP002540627 ISSN: 0022-3549

## Description

The present invention relates to a solid pharmaceutical composition superior in stability and dissolution property, which comprises a low viscosity binder.

### Background of the Invention

It is needless to say that pharmaceutical products are required to have effectiveness and safety. To secure effectiveness and safety of a pharmaceutical product, not only the effectiveness and safety of the active ingredient but also the properties from the aspect of manufacturing pharmacy such as stability of the active ingredient in the preparation, dissolution property of the drug from the preparation and the like are extremely important. For example, even if a preparation satisfies a certain level of quality immediately after production, if the active ingredient in the preparation decomposes over time, the preparation is problematic in terms of effectiveness and safety as a pharmaceutical product. As to the dissolution property of the drug from the preparation, when dissolution of the drug from the preparation is too slow, the drug in blood may fail to reach an effective concentration and an expected efficacy may not be achieved. Conversely, when dissolution of the drug from the preparation is too fast, the drug concentration in blood may rapidly increase and the risk of side effects may also increase.

As a method for increasing the stability of the active ingredient in a preparation, addition of a fat and oil-like substance having a low melting point is known. For example, a compound represented by the formula (I) (e.g., benzimidazole-7-carboxylic acid derivative and the like) having a strong angiotensin II receptor antagonistic action and useful as a therapeutic drug for hypertension and the like is a crystalline compound stable to temperature, humidity, heat etc. when it is a single solid compound. However, distortion of crystal due to the pressure, friction, heat and the like applied in granulation or compression during the production process often occurs and decrease in the content with time is accelerated. Decomposition with time of a preparation is known to be suppressed by adding a fat and oil-like substance having a low melting point (patent reference 1: JP-A-5-194218).

On the other hand, in the field of pharmaceutical products, plural preparations containing the same active ingredient at varying drug contents are often sold for the purpose of controlling the dose depending on the severity of the disease and the like. In order to exhibit the efficacy comparable to the content and secure safety in this case, the drug dissolution rate from the preparation needs to be constant irrespective of drug contents. However, it is known that the disintegratability of tablets decreases because tablet weight increases as the scale of tablet increases, and the dissolution property of the drug decreases. Since dissolution of drug from a solid dosage form is correlated with the disintegratability of the solid dosage form, as a method for improving the drug dissolution property from a solid dosage form, the kind and addition method of a disintegrant are generally changed.

### Disclosure of the Invention

When a fat and oil-like substance having a low melting point was added to improve the stability of the active ingredient in a solid dosage form, disintegratability of the solid dosage form was degraded, the dissolution property of the drug from the solid dosage form decreased markedly. Particularly, degradation of the drug dissolution property was remarkable as the content of the active ingredient in a solid dosage form increased. While the present inventors studied various kinds and addition methods of a disintegrant, the dissolution rate could not be improved.

Therefore, the present inventors have conducted intensive studies in an attempt to improve the drug dissolution property of a solid dosage form containing a fat and oil-like substance having a low melting point and found that the drug dissolution property from the solid dosage form can be unexpectedly improved by the addition of a low viscosity binder to a preparation, which resulted in the completion of the present invention.

Accordingly, the present invention relates to
(1) a solid pharmaceutical composition defined according to appended claim 1;
(2) composition of the aforementioned (1), which is a tablet;
(3) a method of improving dissolution of a compound, as defined in appended claim 4.

In the composition and method of the invention, the crystalline compound or a salt thereof is 2-ethoxy-1-{[2'-(5-oxo-4,5-dihydro-1,2,4-oxadiazol-3-yl)biphenyl-4-yl]methyl}-1H-benzimidazole-7-carboxylic acid (hereinafter sometimes to be abbreviated as compound A).

### Brief Description Of The Drawings

Fig. 1 is a graph showing the dissolution ratios of the Example and Reference Examples.
Fig. 2 is a graph showing the dissolution ratios of the Example and Reference Examples.

As salts of compound A, pharmaceutically acceptable salts can be mentioned and, for example, salts of compound A with inorganic base, salts thereof with organic base, salts thereof with inorganic acid, salts thereof with organic acid, salts thereof with basic or acidic amino acid can be mentioned. As preferable examples of the salts with inorganic base, for example, alkali metal salts such as sodium salt, potassium salt; alkaline earth metal salts such as calcium salt, magnesium salt; aluminum salt, ammonium salt can be mentioned. As preferable examples of salts with the organic base, for example, salts with trimethylamine, triethylamine, pyridine, picoline, ethanolamine, diethanolamine, triethanolamine, dicyclohexylamine, N,N'-dibenzylethylenediamine can be mentioned. As preferable examples of the salts with inorganic acid, for example, salts with hydrochloric acid, hydrobromic acid, nitric acid, sulfuric acid, phosphoric acid can be mentioned. As preferable examples of the salts with organic acid, for example, salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid can be mentioned. As preferable examples of the salts with basic amino acid, for example, salts with arginine, lysine, ornithine can be mentioned, and as preferable examples of the salts with acidic amino acid, for example, salts with aspartic acid, glutamic acid can be mentioned.

In the production of the pharmaceutical composition of the present invention, the polyethylene glycol can be added uniformly with the active ingredient as compared to substances like fat and oil having a high melting point and, as a result, a more stable pharmaceutical composition with suppressed decomposition of the active ingredient can be obtained.

Polyethylene glycols having a molecular weight of from 1,000 to 10,000 (e.g., polyethylene glycol 6000 (Macrogol 6000) etc.) are used in the composition and method of the invention. These fat and oil-like substances having a low melting point may be used alone or in a combination of two or more kinds thereof.

As the hydroxypropylcellulose (HPC) to be used in the present invention, a HPC having a viscosity of 1 - 4 mPa·s, as measured at 20°C using a 2% aqueous solution model B viscometer (Brookfield-type viscometer) is used.

Hydroxypropylcellulose of this type is commercially available, for example, as products such as NIPPON SODA CO., LTD. SSL grade, SL grade.

As the solid pharmaceutical composition of the present invention, for example, a solid dosage form suitable for oral administration such as tablet, granule, fine granules, capsule, pill can be mentioned, with preference given to tablet.

The solid dosage form can be produced by a method known per se (e.g., the method described in the Japanese Pharmacopoeia 14th Edition, General Principles). For example, a tablet can be produced by incorporating the hydroxypropylcellulose and the polyethylene glycol.
into the active ingredient, followed by subjecting the mixture to molding. The incorporation is conducted by a method conventionally employed in the field of pharmaceutical preparations, such as mixing, kneading, massing, sieving, stirring and the like. For example, the hydroxypropylcellulose, the active ingredient and the polyethylene glycol may be directly mixed (addition in a powder state), or a solvent is added to the mixture, followed by conventional kneading, granulating and drying. Alternatively, the polyethylene glycol and the hydroxypropylcellulose are dissolved in a suitable solvent, then the solution is uniformly mixed with the active ingredient, followed by conventional kneading, granulating and drying (addition in a liquid state). Furthermore, a liquid material containing the hydroxypropylcellulose and the polyethylene glycol and a liquid material containing the active ingredient can be independently sprayed onto a powder material such as an excipient, followed by mixing the resultant material. In the case of "addition in a liquid state", any solvent which does not exert undesirable influence on the active ingredient, for example, water, dimethylformamide, acetone, ethanol, propyl alcohol, isopropyl alcohol, butyl alcohol, methylene chloride, trichloroethane etc., can be employed. After completion of blending, the material is subjected to a conventional molding process under pressurization to prepare tablets containing the active ingredient. The molding under pressurization means that a material is compressed under pressurization into a desired form, which most generally refers to tabletting.

It is also possible to add a variety of additives to be employed for preparation making to the solid pharmaceutical composition of the present invention in an adequate step. For example, excipients such as crystalline cellulose (e.g. Avicel PH 101 (manufactured by Asahi Chemical Industry Co., Ltd.)), carboxymethyl cellulose calcium, corn starch, wheat starch, lactose, sucrose, glucose, calcium sulfate, calcium phosphate, sodium chloride etc., binders such as gum arabic, gelatin, methyl cellulose, polyvinyl pyrrolidone, hydroxypropyl cellulose (hereinafter sometimes abbreviated as HPC), hydroxypropylmethyl cellulose etc., lubricants such as magnesium stearate, talc, synthetic aluminum silicate, sodium lauryl sulfate, boric acid, magnesium oxide, paraffin etc., colorants, flavoring agents, odor-improving agents.

Furthermore, the solid pharmaceutical composition of the present invention can also be prepared into coated tablets. The coating may be conducted by a method known per se. As the coating agents, conventional coating agents (e.g. hydroxypropylmethyl cellulose, hydroxypropyl cellulose, methyl cellulose, polyvinyl pyrrolidone etc.), and as auxiliary agents for coating, use is made of, for example, polyethylene glycol 6000, polysorbate (e.g. Tween 80 etc.), titanium oxide, and pigments such as red iron oxide can be used.

The solid pharmaceutical composition of the present invention contains a hydroxypropylcellulose in a proportion of (coated tablet is without coating) 0.5 - 15 wt%, preferably 1 - 10 wt%, more preferably 2 - 5 wt%, in the composition. The polyethylene glycol is contained in a proportion of (coated tablet is without coating) 0.5 - 15 wt%, preferably 1 - 10 wt%, more preferably 2 - 5 wt%, in the composition. The active ingredient is contained in a proportion of (coated tablet is without coating) 0.1 - 40 wt%, preferably 1 - 30 wt%, more preferably 2 - 25 wt%, in the composition. The content of the active ingredient is about 1 - about 150 mg, preferably about 2 - about 100 mg, more preferably about 2 - about 80 mg.

From the aspect of disintegratability, the solid pharmaceutical composition of the present invention preferably disintegrates within 30 min in an aqueous solution. The solid pharmaceutical composition of the present invention thus-obtained by adding the polyethylene glycol and the hydroxypropylcellulose to the active ingredient suppresses decomposition with time due to molding and becomes a clinically extremely useful preparation superior in the dissolution property.

The solid pharmaceutical composition of the present invention can be safely administered as a pharmaceutical agent for a mammal (e.g., human, dog, rabbit, rat, mouse and the like).

The dose of a particular patient is determined in consideration of the age, body weight, general health condition, sex, diet, administration time, clearance rate, drug combination and the like, as well as the severity of the disease for which the patient is undergoing the treatment. The daily dose is about 0.05 - 500 mg, preferably 0.1 - 100 mg, as a compound represented by the formula (I).

### Examples

The present invention is explained in more detail in the following by referring to Examples and Reference Examples, which are not to be construed as limitative.

Compound A is 2-ethoxy-1-{[2'-(5-oxo-4,5-dihydro-1,2,4-oxadiazol-3-yl)biphenyl-4-yl]methyl}-1H-benzimidazole-7-carboxylic acid (melting point: 191°C, solubility in water at 20°C of about 0.006 g/L). In the following Examples and Reference Examples, the Japanese Pharmacopoeia 14th Edition or Japanese Pharmaceutical Excipients 2003 compatible products were used as the preparation additives. Of the preparation additives, while magnesium stearate is also the Japanese Pharmacopoeia 14th Edition compatible product, like other preparation additives, it particularly has a stearic acid content ratio of not less than about 90% (Taihei Chemical Industrial Co., Ltd.).

### Example 1

Using a fluidized bed granulator (POWREX, Lab-1) and according to the following formulation (Table 1), compound A obtained in Reference Example 4, lactose and cornstarch were mixed, and an aqueous solution of polyethylene glycol 6000 as a fat and oil-like substance having a low melting point in hydroxypropylcellulose (viscosity 2 - 3.4 mPa_{·}s) was sprayed as a binder liquid, granulated, dried and sized. Low-substituted hydroxypropylcellulose and magnesium stearate were added and mixed, and the mixture was tabletted using a tabletting machine (Shimadzu Corporation, AUTOGRAPH AG-1) with a 8.0 mmφ biconvex punch at weight 200 mg, pressure 8.5 kN.

### Example 2

Using a fluidized bed granulator (POWREX, Lab-1) and according to the following formulation (Table 1), compound A obtained in Reference Example 4, lactose and cornstarch were mixed, and an aqueous solution of polyethylene glycol 6000 as a fat and oil-like substance having a low melting point in hydroxypropylcellulose (viscosity 2 - 3.4 mPa·s) was sprayed as a binder liquid, granulated, dried and sized. Low-substituted hydroxypropylcellulose and magnesium stearate were added and mixed, and the mixture was tabletted using a tabletting machine (Shimadzu Corporation, AUTOGRAPH AG-1) with a 13 mm×8 mm oval type convex punch at weight 400 mg, pressure 10.5 kN.

### Example 3

Using a fluidized bed granulator (POWREX, FD-5S) and according to the following formulation (Table 2), compound A obtained in Reference Example 5, lactose and cornstarch were mixed, and an aqueous solution of polyethylene glycol 6000 as a fat and oil-like substance having a low melting point in hydroxypropylcellulose (viscosity 2 - 3.4 mPa·s) was sprayed as a binder liquid, granulated, dried and sized. Low-substituted hydroxypropylcellulose, crystalline cellulose and magnesium stearate were added and mixed, and the mixture was tabletted using a tabletting machine (KIKUSUI SEISAKUSHO LTD., Correct 19K) with a 7 mmφ biconvex punch at weight 130 mg, pressure 7 kN.

### Example 4

Using a fluidized bed granulator (POWREX, FD-5S) and according to the following formulation (Table 2), compound A obtained in Reference Example 5, lactose and cornstarch were mixed, and an aqueous solution of polyethylene glycol 6000 as a fat and oil-like substance having a low melting point in hydroxypropylcellulose (viscosity 2 - 3.4 mPa·s) was sprayed as a binder liquid, granulated, dried and sized. Low-substituted hydroxypropylcellulose, crystalline cellulose and magnesium stearate were added and mixed, and the mixture was tabletted using a tabletting machine (KIKUSUI SEISAKUSHO LTD., Correct 19K) with a 8.5 mmφ biconvex punch at weight 260 mg, pressure 8 kN.

### Example 5

Using a fluidized bed granulator (POWREX, FD-5S) and according to the following formulation (Table 2), compound A obtained in Reference Example 5, lactose, cornstarch and crystalline cellulose were mixed, and an aqueous solution of polyethylene glycol 6000 as a fat and oil-like substance having a low melting point in hydroxypropylcellulose (viscosity 2 - 3.4 mPa·s) was sprayed as a binder liquid, granulated, dried and sized. Low-substituted hydroxypropylcellulose, crystalline cellulose and magnesium stearate were added and mixed, and the mixture was tabletted using a tabletting machine (KIKUSUI SEISAKUSHO LTD., Correct 19K) with a 7 mmφ biconvex punch at weight 130 mg, pressure 7 kN.

### Example 6

Using a fluidized bed granulator (POWREX, FD-5S) and according to the following formulation (Table 2), compound A obtained in Reference Example 5, lactose, cornstarch and crystalline cellulose were mixed, and an aqueous solution of polyethylene glycol 6000 as a fat and oil-like substance having a low melting point in hydroxypropylcellulose (viscosity 2 - 3.4 mPa·s) was sprayed as a binder liquid, granulated, dried and sized. Low-substituted hydroxypropylcellulose, crystalline cellulose and magnesium stearate were added and mixed, and the mixture was tabletted using a tabletting machine (KIKUSUI SEISAKUSHO LTD., Correct 19K) with a 8.5 mmφ biconvex punch at weight 260 mg, pressure 7 kN.

### Reference Example 1

Using a fluidized bed granulator (POWREX, FD-5S) and according to the following formulation, compound A obtained in Reference Example 4, lactose and cornstarch were mixed, and an aqueous solution of polyethylene glycol 6000 as a fat and oil-like substance having a low melting point in hydroxypropylcellulose (viscosity 6 - 10 mPa·s) was sprayed as a binder liquid, granulated, dried and sized. Low-substituted hydroxypropylcellulose and magnesium stearate were added and mixed, and the mixture was tabletted using a tabletting machine (KIKUSUI SEISAKUSHO LTD., Correct 19K) with a 6.5 mmφ biconvex punch at weight 100 mg, pressure 7 kN.

### Reference Example 2

Using a fluidized bed granulator (POWREX, Lab-1) and according to the following formulation, compound A obtained in Reference Example 4, lactose and cornstarch were mixed, and an aqueous solution of polyethylene glycol 6000 as a fat and oil-like substance having a low melting point in hydroxypropylcellulose (viscosity 6 - 10 mPa·s) was sprayed as a binder liquid, granulated, dried and sized. Low-substituted hydroxypropylcellulose and magnesium stearate were added and mixed, and the mixture was tabletted using a tabletting machine (Shimadzu Corporation, AUTOGRAPH AG-1) with a 8.0 mmφ biconvex punch at weight 200 mg, pressure 8.5 kN.

### Reference Example 3

Using a fluidized bed granulator (POWREX, Lab-1) and according to the following formulation, compound A obtained in Reference Example 4, lactose and cornstarch were mixed, and an aqueous solution of polyethylene glycol 6000 as a fat and oil-like substance having a low melting point in hydroxypropylcellulose (viscosity 6 - 10 mPa·s) was sprayed as a binder liquid, granulated, dried and sized. Low-substituted hydroxypropylcellulose and magnesium stearate were added and mixed, and the mixture was tabletted using a tabletting machine (Shimadzu Corporation, AUTOGRAPH AG-1) with a 13 min×8 mm oval type convex punch at weight 400 mg, pressure 10.5 kN.

### Reference Example 4

To methyl 2-ethoxy-1-{[2'-(5-oxo-4,5-dihydro-1,2,4-oxadiazol-3-yl)biphenyl-4-yl]methyl}-1H-benzimidazole-7-carboxylate (10 g) was added 0.40N-NaOH (167 mL) and the mixture was stirred at 65-75°C for 1-1.5 hr. The mixture was adjusted to pH 8 at room temperature with 1N HCl, activated carbon (0.5 g) was added and the mixture was stirred. The activated carbon was filtered off and the residue was washed with water (17 mL). The mixture was adjusted to pH 3 with 1N HCl at 0-5°C. The mixture was stirred at 40-45°C and then at 0-10°C. The precipitated crystals were collected by filtration, washed with water (17 mLx2 times), and dried at 40°C to give compound A as a white powder (9.3 g, yield 96%).

### Reference Example 5

To methyl 2-ethoxy-1-{[2'-(5-oxo-4,5-dihydro-1,2,4-oxadiazol-3-yl)biphenyl-4-yl]methyl}-1H-benzimidazole-7-carboxylate (10 g) was added 0.36N-NaOH (150 mL) and the mixture was stirred at 65-75°C for 1.5 hr. The mixture was adjusted to pH 8 at room temperature with 1N HCl, activated carbon (0.5 g) was added and the mixture was stirred. The activated carbon was filtered off and the residue was washed with water (50 mL). The mixture was adjusted to pH 3 with 0.5N HCl at 9-15°C. The mixture was stirred at 40-45°C and then at 5-15°C. The precipitated crystals were collected by filtration, washed with water (20 mL), and dried at 40°C to give compound A as a white powder (9.3 g, yield 96%).

### Experimental Example

The tablets obtained in Examples 1, 2 and Reference Examples 1, 2, 3 were subjected to a test according to the Dissolution Test Method 2 (Paddle Method, 50 rpm, 37°C) and using phosphate buffer, pH 6.8/water mixture (1:1), 900 mL, as a test solution.

**Table 1**

| composition | Ref. Ex. 1 | Ref. Ex. 2 | Ref. Ex. 3 | Ex. 1 | Ex. 2 |
|---|---|---|---|---|---|
| compound A | 10.0 | 20.0 | 40.0 | 20.0 | 40.0 |
| lactose | 51.0 | 102.0 | 204.0 | 102.0 | 204.0 |
| cornstarch | 23.0 | 46.0 | 92.0 | 46.0 | 92.0 |
| hydroxypropylcellulose (viscosity 6-10 mPa·s) | 3.0 | 6.0 | 12.0 | - | - |
| hydroxypropylcellulose (viscosity 2-3.4 mPa·s) | - | - | - | 6.0 | 12.0 |
| macrogol 6000 | 3.0 | 6.0 | 12.0 | 6.0 | 12.0 |
| low-substituted hydroxypropylcellulose | 9.5 | 19.0 | 38.0 | 19.0 | 38.0 |
| magnesium stearate | 0.5 | 1.0 | 2.0 | 1.0 | 2.0 |
| total | 100.0 | 200.0 | 400.0 | 200.0 | 400.0 |

**Table 2**

| composition | Ex. 3 | Ex. 4 | Ex. 5 | Ex. 6 |
|---|---|---|---|---|
| compound A | 20 | 40 | 40 | 80 |
| lactose | 53.9 | 107.8 | 29.3 | 58.6 |
| cornstarch | 20 | 40 | 13 | 26 |
| hydroxypropylcellulose (viscosity 2-3.4 mPa·s) | 4 | 8 | 4 | 8 |
| macrogol 6000 | 4 | 8 | 4 | 8 |
| low-substituted hydroxypropylcellulose | 12.4 | 24.8 | 13 | 26 |
| crystalline cellulose | 15 | 30 | 26 | 52 |
| magnesium stearate | 0.7 | 1.4 | 0.7 | 1.4 |
| total | 130 | 260 | 130 | 260 |
| shape | 7 mmφ | 8.5 mmφ | 7 mmφ | 8.5 mmφ |

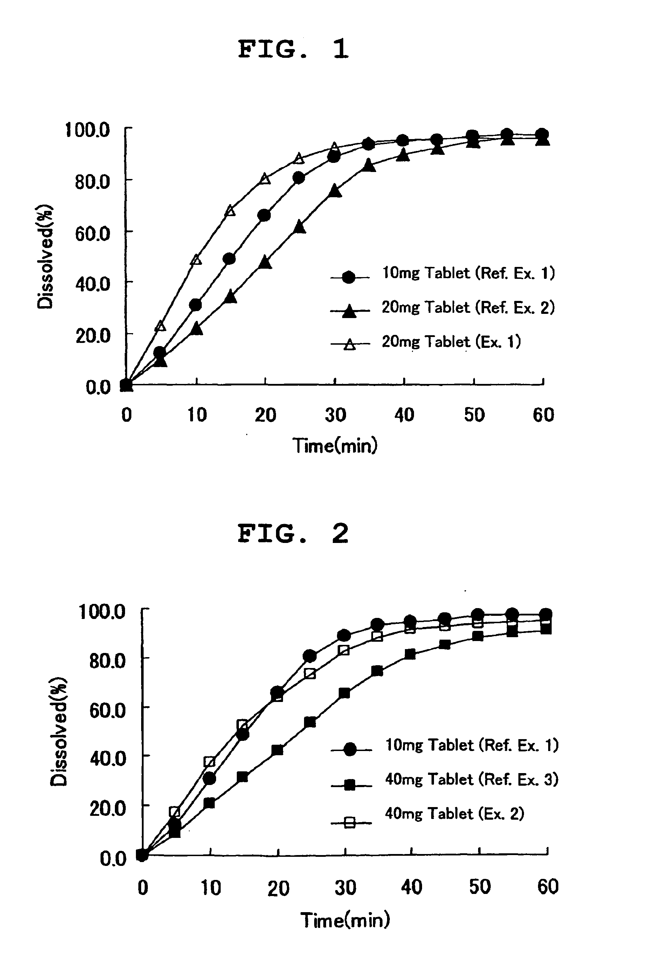

As shown in Figs. 1 and 2, a tablet containing a low viscosity binder shows superior dissolution property as compared with a tablet containing a binder having a conventional viscosity, and by the addition of a low viscosity binder, the drug dissolution property could be easily controlled.

### Industrial Applicability

Since the solid pharmaceutical composition of the present invention is simultaneously superior in the stability and dissolution property, it is extremely useful as a pharmaceutical product preparation technique.

## Claims

1. A solid pharmaceutical composition comprising crystalline 2-ethoxy-1-{[2'-(5-oxo-4,5-dihydro-1,2,4-oxadiazol-3-yl)biphenyl-4-yl]methyl}-1H-benzimidazole-7-carboxylic acid or a salt thereof, polyethylene glycol having a molecular weight of from 1,000 to 10,000 and a melting point of 20°C - 90°C and hydroxypropylcellulose having a viscosity of 1 - 4 mPa·s, as measured at 20°C using a 2% aqueous solution Brookfield-type viscometer.

2. The composition of claim 1, which further comprises crystalline cellulose.

3. The composition of claim 1, which is a tablet.

4. A method of improving dissolution of a compound which is crystalline 2-ethoxy-1-{[2'-(5-oxo-4,5-dihydro-1,2,4-oxadiazol-3-yl)biphenyl-4-yl]methyl}-1H-benzimidazole-7-carboxylic acid or a salt thereof from a solid pharmaceutical composition comprising the compound or a salt thereof and polyethylene glycol having a molecular weight of from 1,000 to 10,000 and a melting point of 20°C - 90°C, which comprises using hydroxypropylcellulose having a viscosity of 1 - 4 mPa·s, as measured at 20°C using a 2% aqueous solution Brookfield-type viscometer.

## Patentansprüche

1. Feste pharmazeutische Zubereitung, die kristalline 2-Ethoxy-1-{[2'-(5-oxo-4,5-dihydro-1,2,4-oxadiazol-3-yl)biphenyl-4-yl]methyl}-1H-benzimidazol-7-carbonsäure oder ein Salz davon, Polyethylenglycol mit einem Molekulargewicht von 1000 bis 10000 und einem Schmelzpunkt von 20 bis 90 °C und Hydroxypropylcellulose mit einer Viskosität von 1 bis 4 mPa·s aufweist, und zwar bei 20 °C unter Verwendung einer 2 %igen wässrigen Lösung mit einem Viskosimeter vom Brookfield-Typ gemessen.

2. Zubereitung nach Anspruch 1,
die ferner kristalline Cellulose aufweist.

3. Zubereitung nach Anspruch 1,
die eine Tablette ist.

4. Verfahren zum Verbessern des Auflösens einer Verbindung, die kristalline 2-Ethoxy-1-{[2'-(5-oxo-4,5-dihydro-1,2,4-oxadiazol-3-yl)biphenyl-4-yl]methyl}-1H-benzimidazol-7-carbonsäure oder ein Salz davon ist, aus einer festen pharmazeutischen Zubereitung, die diese Verbindung oder ein Salz davon und Polyethylenglycol mit einem Molekulargewicht von 1000 bis 10000 und einem Schmelzpunkt von 20 bis 90 °C aufweist,
das die Verwendung von Hydroxypropylcellulose mit einer Viskosität von 1 bis 4 mPa·s aufweist, und zwar bei 20 °C unter Verwendung einer 2 %igen wässrigen Lösung mit einem Viskosimeter vom Brookfield-Typ gemessen.

## Revendications

1. Composition pharmaceutique solide, comprenant de l'acide 2-éthoxy-1-{[2'-(5-oxo-4,5-dihydro-1,2,4-oxadiazol-3-yl)-biphényl-4-yl]-méthyl}-1H-benzimidazole-7-carboxylique ou un sel de cet acide, à l'état cristallin, un polyéthylèneglycol présentant une masse moléculaire de 1000 à 10000 et un point de fusion de 20 à 90 °C, et une hydroxypropyl-cellulose présentant une viscosité, mesurée à 20 °C, pour une solution aqueuse à 2 % et à l'aide d'un viscosimètre de type Brookfield, de 1 à 4 mPa.s.

2. Composition conforme à la revendication 1, qui comporte en outre de la cellulose cristalline.

3. Composition conforme à la revendication 1, qui est un comprimé.

4. Procédé permettant d'améliorer la dissolution d'un composé, qui est de l'acide 2-éthoxy-1-{[2'-(5-oxo-4,5-dihydro-1,2,4-oxadiazol-3-yl)-biphényl-4-yl]-méthyl}-1H-benzimidazole-7-carboxylique, ou un sel de ce composé, à l'état cristallin, à partir d'une composition pharmaceutique solide comprenant ce composé, ou l'un de ses sels, et un polyéthylèneglycol présentant une masse moléculaire de 1000 à 10000 et un point de fusion de 20 à 90 °C, lequel procédé comporte le fait d'utiliser une hydroxypropyl-cellulose présentant une viscosité, mesurée à 20 °C, pour une solution aqueuse à 2 % et à l'aide d'un viscosimètre de type Brookfield, de 1 à 4 mPa.s.
